# EUROPEAN PATENT SPECIFICATION

(11) **EP 1 086 701 B1**
(45) Date of publication and mention of the grant of the patent: **08.03.2006**
(21) Application number: 00307689.0
(22) Date of filing: 06.09.2000
(51) Int. Cl.: A61K 36/00, A61K 31/80, A61P 1/10, A61K 31/44

(54) **Simethicone containing laxative composition**
Simethicon enthaltendes Abführmittel
Composition laxative contenant de la siméthicone

(30) Priority: 07.09.1999 US 390813
(43) Date of publication of application: 28.03.2001
(73) Proprietor: McNEIL-PPC, INC., Skillman, NJ 08858 (US)
(72) Inventor: McNally, Gerard P., Strafford, PA 19087 (US); Pendley, Charles E., Abington, PA 19001 (US)
(74) Representative: Mercer, Christopher Paul

(56) References cited:
- FR-A- 2 017 735
- FR-M- 5 549
- HELDWEIN W ET AL: "Evaluation of the usefulness of dimethicone and/or senna extract in improving the visualization of abdominal organs." JOURNAL OF CLINICAL ULTRASOUND, (1987 SEP) 15 (7) 455-8. , XP008009977

## Description

### Field of the Invention

The present invention relates to a laxative composition, more particularly a laxative composition containing a therapeutic amount of simethicone or dimethicone.

### Background of the Invention

Laxative compositions are typically categorized by the mechanism in which they work, such as bulk, saline, stool softener, lubricant, or stimulant as per the U.S. Food and Drug Administration's monograph, Laxatives, Martindale, page 1070; or Goodman and Gilman page 914. Bulk laxatives contain materials such as psyllium, cellulose, polycarbophil, bran, karaya and malt soup extract. Saline laxatives, such as magnesium, hydroxide, sulfate, phosphate, and citrate salts act by drawing water into the intestines. Stool softeners include docusate salts and mineral oils. Lubricant laxatives include mineral oil, and certain digestible plant oils. Lubricants coat the fecal contents, preventing excess absorption of water in the colon. Stimulants include bisacodyl, cascara sagrada, senna, aloe, castor oil and dehydrocholic acid. Stimulant laxatives work to increase intestinal motility by either increasing peristaltic activity as a result of local irritation, or by selective stimulation of the nerves which activate intestinal smooth muscle.

In addition to the above compounds, US Pat. No. 5,418,220 discloses simethicone as a treatment for constipation. In the patent example, one teaspoon of a dimethicone suspension (approximately 33% in glycerin stearate and water) resulted in laxation in a 2 year old patient approximately two hours after administration.

While the above materials are effective laxative materials, there is a continuing need to enhance the performance of these materials by providing faster onset of action and superior efficacy.

### Summary of the Invention

The present invention provides a composition comprising:
a) a laxative; and
b) simethicone for enhancing the efficacy of the laxative; characterised in that the laxative is bisacodyl.

In a second embodiment, the invention provides the use of a composition as defined herein, in the manufacture of a medicament for: treating constipation; improving gastro-intestinal motility; treating diabetic gastro-paresis; or treating gastro-esophogeal reflux disorder.

### Detailed Description of the Invention

Dimethicone is a well known pharmaceutical material consisting of linear siloxane polymers containing repeating units of the formula {-(CH₂)₂SiO}ₙ stabilized with trimethylsiloxy end blocking units of the formula [(CH₃)₃SiO-]. Simethicone is the mixture of dimethicone and silicon dioxide. The level of simethicone in the present invention is sufficient to enhance the effect of a stimulant laxative agent bisacodyl. Generally this level is from about 0.1 mg to about 500 mg, preferably from about 1 to about 500 mg, and most preferably from about 20 mg to about 125 mg per dosage unit. A dosage unit can be one tablet, capsule, or suppository, one teaspoonful of a liquid, or one single portion of any other sutiable delivery form. The level of laxative is the amount necessary to provide the desired effect, which is generally from about 1.0 mg to about 100mg. preferably from about 1.0 mg to about 50 mg, and most preferably from about 1.0mg to about 15 mg per dose for bisacodyl.

The present invention can be delivered as a tablet, a chewable tablet, a liquid drink, a suppository or other pharmaceutically acceptable forms. Oral delivery forms are preferred.

Commonly known pharmaceutically acceptable additives for orally-administered drugs such as enteric polymers, taste-masking polymers, binders, sweeteners, flavoring agents, dispersants, buffering agents and the like may be included in amounts that do not adversely affect the novel properties of the formulation described and claimed herein. Suitable enteric polymer systems include polymethacryaltes (e.g. EUDRAGIT L30D or S100, available from Rohm Company); cellulose acetate phthalate; polyvinyl acetate phthalate, hydroxypropyl methylcellulose phthalate. Suitable binders include microcrystalline cellulose, calcium phosphates, dextrates. Suitable dispersants include croscarmellose sodium, methylcellulose, hydroxymethylcellulose, hydroxypropylmethylcellulose, hydroxyethylcellulose and the like. Suitable sweeteners include sugar, sorbitol, saccharin, mannitol, glucose, aspartame, sucralose and the like. Flavoring agents include peppermint, spearmint, cinnamon, vanilla and the like. A more complete listing of appropriate additives can be found in numerous publications including *Remington's Encylcopedia.*

The present invention is surprising and unexpected in that PCT EP95/00973 previously disclosed that simethicone is effective in association or affinity to the surface structure of the GI tract. The PCT patent application continues that due to the increased adhesion properties of dimethicone the residence time of an active ingredient in a region of the GI tract can be substantially prolonged if dimethicone is used as a transport or carrier system.

As used herein diabetic gastro-paresis is defined as the dilation of the stomach with gastric retention seen in diabetics, commonly seen in association with severe acidosis or coma, Stedmans Medical Dictionary. Gastro-esophogeal reflux disorder (GERD) is defined as the regurgitation of the contents of the stomach into the esophagus, possibly into the pharynx where they can be aspirated between the vocal cords and down into the trachea; providing symptoms of burning pain and acid taste result; pulmonary complications of aspirations are dependent upon the amount content and acidity of the aspirate. Id.

The following examples are provided to further illustrate the claimed invention, but not limit the invention to the examples provided below.

### Example 1

A study was performed to characterize and compare the effects of bisacodyl, simethicone and combinations of bisacodyl and simethicone on small bowel transit time. Small intestinal transit time was studied as a surrogate for laxation in the rat. Leng-Peschlow, E., "Effect of Sennosides A+B and Bisacodyl on rat large intestine", Pharmacology, vol. 38 (1989), 310-318 (1989). observed increased fecal output over the range of 10-100mg/kg of bisacodyl given orally, large intestinal transit was significantly stimulated by 50 mg/kg bisacodyl given orally. The prokinetic efficacy of bisacodyl in the small intestine may differ from that observed in the large intestine by Leng-Psechlow. For the small intestinal transit model a sub-therapeutic dose of 25mg/kg bisacodyl was chosen.

Rats were dosed with a suspension of powdered charcoal which served as a non-absorbable marker. The rats were also dosed with bisacodyl USP 23 (ZetaPharm, Inc.) and simethicone supplied as MYLICON® drops and activated charcoal (Sigma Chemical). Six treatments were compared a control; simethicone 15 mg/kg; bisacodyl 25 mg/kg; and bisacodyl 25 mg/kg and simethicone at 5, 10 or 15 mg/kilogram.

Dosing preparations of charcoal suspension (10 weight percent) were made freshly each day by adding dry powder to 0.5 percent methylcellulose in water and stirring. Dosing preparations were made each day by adding the appropriate quantity of bisacodyl and / or simethicone drops to the charcoal suspension. All treatments were administered orally, using the dose volume of 10 milliliters per kilogram.

Sixty minutes after dosing, the rats were sacrificed and small bowel transit rate was determined by identifying the charcoal marker and measuring its distance from the pylorus.

Results reveal that small bowel motility was greater in rats treated with bisacodyl and simethicone combinations than in rats treated with either bisacodyl or simethicone alone. The results are presented below.

| **Treatment** | **Mean % traveled** | **Std. Error of Mean** | **% Increase** |
|---|---|---|---|
| Vehicle Control | 79.9 | 2.1 | -- |
| Simethicone 15mg/kg | 79.1 | 2.0 | -1 |
| Bisacodyl 25 mg/kg | 80.7 | 2.0 | 1 |
| Bisacodyl 25 mg/kg + Simethicone 5 mg/kg | 90.3 | 2.5 | 13 |
| Bisacodyl 25 mg/kg + Simethicone 10 mg/kg | 97.0 | 1.7 | 21 |
| Bisacodyl 25 mg/kg + Simethicone 15 mg/kg | 94.4 | 2.0 | 18 |

The results reveal that although small bowel transit was not different from control in rats treated with simethicone alone or bisacodyl alone, small bowel transit definitely increased in rats treated with the combination. The observed increases in small bowel transit were sizable (13 to 21 percent increase compared to the control) and the result was also statistically significant (p less than 0.05).

### Example 2

### Preparation of Chewable Tablets Containing Bisacodyl and Simethicone

### PART A.

1) 700 gm of granular tricalcium phosphate (Tritab®, Rhone-Poulenc, Shelton, Ct) is added to the mixing bowl of a Kitchen Aid mixer.
2) While mixing at low speed, over a period of 5 minutes add 200 gm of simethicone, USP.
3) Continue mixing at low speed for an additional 5 minutes.
4) Add 2.5 gm of silicon dioxide and mix an additional 5 minutes.

### PART B.

Preparation of enteric coated bisacodyl particles.
1. Rotogranulation.
   Combine 0.52 kg. of Bisacodyl, 0.24 kg. of Hydroxypropyl Methylcellulose ( grade Methocel E5) and 3.24 kg. of Lactose impalpable in a rotor granulator bowl. Rotor granulate by spraying water (approx. 1.0 kg.) at a rotor speed of 500 RPM. Dry the rotogranulated particles to a product temperature of 30°-35°C after decreasing rotor speed to 250 RPM.
2. Particle Coating.
   Coat the particles produced in Step A in a Wurster Coating apparatus. The polymer coating solution should consist of the following; approximately 35% by weight aq. dispersion of Eudragit L30D containing approx. 2.5% Triethyl citrate. Apply 10-40% by weight polymer to the particles. Maintain product temperature at about 30-32°C during the coating step. A further tastemasking coat is then applied as follows. The polymer coating solution should consist of 10% by weight solution of cellulose acetate 398-10, (39.8%acetyl content; 10 seconds viscosity) and hydroxypropylcellulose (Klucel EF) where the ratio of CA to HPC is 70/30. The solvent used is an 80/20 mixture of acetone/methanol. Apply a 5-10% by weight polymer coat to the particles. Maintain a product temperature at about 40-42°C during coating.

### PART C.

1) Blend 89 gm of the free flowing granular intermediate from Part A. with 7.34g of coated bisacodyl, 98 gm of Dextrates, 7.5 gm granular sorbitol, 0.6 gm peppermint flavor, and 0.5 gm stearic acid.
2) Compress the blend using 5/8" FFBE tooling to a tablet weight of 1287 mg

### Example 3

### Preparation of Swallowable Film Coated Tablets Containing Bisacodyl and Simethicone.

| **Ingredient** | **Qty (mg/tab)** |
|---|---|
| **PART I** 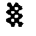 **concentrate** | |
| Dibasic calcium phosphate, NF, Anhydrous, granular (DiTab®) | 500 |
| Simethicone, USP | 125 |
| Tribasic calcium phosphate, NF, Anhydrous, Powder | 25 |
| Dibasic calcium phosphate, NF, Anhydrous, granular Powder (Fujicalin® SG) | 90 |

| **PART II- Bisacodyl/Rxcipient/Binder system** | |
|---|---|
| Bisacodyl, USP | 5 |
| Microcrystalline cellulose, NF (MCC) | 205 |
| croscarmellose sodium, NF | 30 |
| **PART III-Lubricant** | |
| Magnesium Stearate, NF | 4 |

| **PART VI- Enteric Coating Step** | |
|---|---|
| Eudragit® S100 | 140 |

### PART 1)

A concentrate comprised of granular anhydrous dibasic calcium phosphates, and simethicone is prepared by adding simethicone compound, USP to a moving bed of granular dibasic calcium phosphate so that the simethicone is distributed evenly and the granular calcium phosphate particle size remains essentially unchanged. The bed is kept in motion by low shear mixers such as fluid bed, Nauta, PK without intensifier bar, pin mixer, or ribbon mixer. The granulation may then be screened through a No. 20 US Std screen (~ 840 micron).

### PART 2)

Bisacodyl is added with low shear blending until the active ingredient is uniformly dispersed in the Simethicone blend. Excipients including a disintegrant are then added with low shear blending which imparts uniform distribution of the active within a binding matrix of limited compositional range.

### PART 3)

The final addition step is to add a lubricant.
The blend is compressed into tablets using a rotary tablet press.

### PART 4)

Tablets are then enteric film coated in a coating pan with an Eudragit S100 dispersion to a weight increase of approximately 5-25%.

## Claims

1. A composition comprising:
a) a laxative; and
b) simethicone for enhancing the efficacy of the laxative; **characterised in that** the laxative is bisacodyl.

2. The composition of claim 1, wherein the laxative and the simethicone are adapted for oral administration.

3. The composition of claim 1 or claim 2 wherein the amount of the bisacodyl is 1 mg to 15 mg per dose.

4. The composition of any one of claims 1 to 3, wherein the amount of simethicone is from 1 mg to 500 mg per dose.

5. The composition of claim 4, wherein the amount of simethicone is from 20 mg to 125 mg per dose.

6. Use of a composition of any one of claims 1 to 5, in the manufacture of a medicament for: treating constipation; improving gastro-intestinal motility; treating diabetic gastro-paresis; or treating gastro-esophogeal reflux disorder.

## Patentansprüche

1. Zusammensetzung, umfassend:
a) ein Laxativum; und
b) Simethicon zur Verstärkung der Wirksamkeit des Laxativums, **dadurch gekennzeichnet, daß** das Laxativum Bisacodyl ist.

2. Zusammensetzung nach Anspruch 1, wobei das Laxativum und das Simethicon zur oralen Verabreichung geeignet sind.

3. Zusammensetzung nach Anspruch 1 oder Anspruch 2, wobei die Menge an Bisacodyl pro Dosis 1 mg bis 15 mg ist.

4. Zusammensetzung nach einem der Ansprüche 1 bis 3, wobei die Menge an Simethicon pro Dosis von 1 mg bis 500 mg ist.

5. Zusammensetzung nach Anspruch 4, wobei die Menge an Simethicon pro Dosis von 20 mg bis 125 mg ist.

6. Verwendung der Zusammensetzung nach einem der Ansprüche 1 bis 5 zur Herstellung eines Medikaments zur Behandlung von Obstipation, Verbesserung der gastrointestinalen Mobilität, Behandlung von diabetischer Gastroparese, oder Behandlung von gastroösophagealer Refluxkrankheit.

## Revendications

1. Composition comprenant :
a) un laxatif ; et
b) de la siméthicone pour améliorer l'efficacité du laxatif ; **caractérisée en ce que** le laxatif est le bisacodyl.

2. Composition selon la revendication 1, dans laquelle le laxatif et la siméthicone sont adaptés pour l'administration orale.

3. Composition selon la revendication 1 ou la revendication 2, dans laquelle la teneur en bisacodyl est de 1 à 15 mg par dose.

4. Composition selon l'une quelconque des revendications 1 à 3, dans laquelle la teneur en siméthicone est de 1 à 500 mg par dose.

5. Composition selon la revendication 4, dans laquelle la teneur en siméthicone est de 20 à 125 mg par dose.

6. Utilisation d'une composition selon l'une quelconque des revendications 1 à 5, pour la fabrication d'un médicament destiné au traitement de la constipation, à l'amélioration du transit gastro-intestinal, au traitement de la gastro-parésie diabétique ou au traitement des troubles associés au reflux gastro-oesophagien.
